# EUROPEAN PATENT SPECIFICATION

(11) **EP 3 058 945 B1**
(45) Date of publication and mention of the grant of the patent: **14.11.2018**
(21) Application number: 16155637.8
(22) Date of filing: 15.02.2016
(51) Int. Cl.: A61K 31/185, A61K 33/06, A61K 31/19, A61P 17/00, A61P 17/02, A61P 17/04, A61K 31/18, A61K 33/32

(54) **PHARMACEUTICAL COMPOSITIONS AND FORMULATIONS FOR TOPICAL APPLICATION WITH ASTRINGENT AND ANTIMICROBIAL EFFECT**
PHARMAZEUTISCHE ZUSAMMENSETZUNGEN UND FORMULIERUNGEN ZUR TOPISCHEN ANWENDUNG MIT ASTRINGENTER UND ANTIMIKROBIELLER WIRKUNG
COMPOSITIONS PHARMACEUTIQUES ET FORMULATIONS POUR L´APPLICATION TOPIQUE AVEC EFFET ASTRINGENT ET ANTIMICROBIEN

(30) Priority: 19.02.2015 IT MI20150243
(43) Date of publication of application: 24.08.2016
(60) Divisional application: 18197035.1
(73) Proprietor: Hiantis S.r.l., 20146 Milano (IT)
(72) Inventor: ROMANO, Paolo, 20146 Milano (IT)
(74) Representative: Ferreccio, Rinaldo

(56) References cited:
- CN-A- 101 474 421
- JP-A- H07 215 838
- KR-A- 20120 078 810
- US-B1- 7 790 771

## Description

### Field of application

The present invention relates to the pharmaceutical industry field.

In particular, the invention relates to a pharmaceutical formulation based on aluminum acetate endowed with astringent effect and an enhanced antimicrobial action, and a powder composition suitable to produce aqueous dispersions containing aluminum acetate and endowed with astringent effect and an enhanced antimicrobial action.

### Prior art

Aluminum acetate is the key ingredient of Burow's solution, traditionally used since the mid-nineteenth century for its astringent and antibacterial properties.

The Burow's solution is used for the treatment of various skin diseases, including swelling, eczemas, erythemas, insect bites and bruises, and in otology .

The US Pharmacopeia describes a procedure for the preparation of Burow's solution, which comprises the reaction of aluminum sulfate, calcium carbonate and acetic acid, to form basic aluminum acetate and a precipitate of calcium sulfate, which is removed by filtration. The subsequent addition of acetic acid to the filtrate affords the Burow's solution.

In the United States and in some other states, a powder mixture of calcium acetate and aluminum sulfate is marketed, addressed to the preparation of the Burow's solution.

The US patent 2 824 042 describes a composition from which a Burow's solution can be obtained, which is more stable to the storage and the heat, without the need of a filtration step. Said composition comprises dibasic aluminum acetate stabilized with boric acid, acetic acid and an alkali metal acetate.

Such a composition can also comprise a quaternary ammonium chloride or a phenylmercuric salt, which gives to the composition germicidal and deodorizing properties.

The Burow's solution is generally prepared at a concentration of 13%, however, it is often diluted with water before application to concentrations up to 10 times lower.

The Burow's solution at concentrations of 1.3%, 3.25% and 13% has been clinically tested in the treatment of otitis (Thorp MA, Gardiner IB, Prescott CA¹) and it has shown an antibacterial action at all of the three concentrations.

From the data reported in the literature (Thorp MA, Oliver SP, Kruger J, Prescott CA²) it results that the Burow's solution exerts an antibacterial action up to a minimum concentration of 0.08%.

The Burow's solution is used quite widely in the United States, where it is considered by FDA³ as a drug having recognized safety and effectiveness ("GRASE" i.e. "generally recognized as safe and effective"), while in Europe its use is much less frequent and more powerful antiseptics are preferred, even if they are provided with lower or even nil astringent effect.

Actually, the combination of astringent and antibacterial effects of the Burow's solution would be very useful in the treatment of certain skin diseases in which, besides the presence of exudates, there is an actual or potential suprainfection, such as, for example, the eczematous, bullous or ulcerative ones.

KR 20120078810 A discloses an external use skin composition containing dimethyl sulfonyl methane and alum, for the treatment of allergy and inflammation and to promote blood circulation.

JP H07 215838 A discloses a skin external agent compounded with erythritol, a plant galenical and a skin-covering component. The skin external agent contains especially one or more kinds of natural extracts extracted from Eriobotrya japonisaca, Prunus persica, Aloe arborescens, Panax ginseng, and Luffa cylindrica as the plant galenical. The skin external agent contains one or more kinds of fats and oils, a wax, a hydrocarbon or silicone oil, a fatty acid, a higher alcohol, an ester oil, a gum, a polymer having a mol.wt. of ≥ 3000 and a clay mineral as the skin-covering ingredient.

CN 101474421 A discloses a disinfection antiphlogistic bactericidal wet tissue wherein the wet tissue of 500 g contains 0.0001-100 g potassium permanganate. Added with perfume or plant essential oil or peppermint oil, boric acid and ginseng extracts, the wet tissue has the functions of disinfecting, sterilizing, diminishing inflammation, preventing corrosion, relieving itching, preventing infection diffusion, nourishing and protecting skin.

US 7 790 771 concerns the use of tosylchloramide(s), tosylchloramide salt(s), their derivatives and/or the decomposition products for treating diseases of the skin, mucous membranes, organs and tissues, excluding treatment of retroviral diseases (HIV) and disinfecting processes.

A first object of the present invention is to make available a composition such as to provide a solution similar to the Burow's, extemporaneously formed from distinct reagents, and with the same astringent properties but with enhanced antimicrobial action.

A second object is to provide novel pharmaceutical formulations based on aluminum acetate with improved effects compared to analogous formulations of the prior art.

### Summary of the invention

The afore said first object is achieved by providing a powder composition comprising calcium acetate, aluminum sulfate and a third component selected from potassium permanganate (KMnO₄) and chloramine-T (sodium N-chloro-4-toluenesulfonamidate) and optionally at least one cosmetically acceptable excipient.

Preferably the calcium acetate is calcium acetate monohydrate and the aluminum sulfate is aluminum sulfate tetradecahydrate.

In an aspect thereof, such a composition comprises, for one part by weight of calcium acetate monohydrate, 1.41 parts of aluminum sulfate tetradecahydrate and from 0.01 to 0.4 parts of potassium permanganate.

In another aspect thereof, such a composition comprises, for one part by weight of calcium acetate monohydrate, 1.41 parts of aluminum sulfate tetradecahydrate and from 0.025 to 0.75 parts of chloramine-T.

Preferably, the composition consists of calcium acetate monohydrate, aluminum sulfate tetradecahydrate and a third component selected from potassium permanganate and chloramine-T.

The aforementioned compositions may be diluted with water in such proportions to obtain a concentration of aluminum acetate equal to 13% as for the Burow's solution but the dilution with water may be pushed to obtain a concentration of aluminum acetate up to 200 times lower.

By diluting the composition according to the present invention with water slightly veiled aqueous dispersions are obtained with the formation of a fine white deposit as a result of the reaction of the calcium acetate monohydrate with aluminum sulfate tetradecahydrate to give the aluminum acetate. The aqueous dispersion can, however, be used as such, without the need to remove the aforementioned slight deposit.

The aqueous dispersions thus obtained, having a concentration of aluminum acetate equal to at least 0.065% w/w, can be used for the topical treatment of skin conditions selected from the group comprising swelling, hives, eczemas, erythemas, insect bites, bruises, itching, vesicular or bullous exudation, crusted lesions, skin ulcers, atopic dermatitis, herpes zoster, herpes simplex, intertrigo and sunburn.

The aqueous dispersions used for the aforementioned topical treatments have a concentration of aluminum acetate comprised between 0.065% and 13% w/w and preferably between 0.13% and 0.5% w/w.

Obviously, the powder composition according to the present invention can also be prepared using calcium acetate and aluminum sulfate with different degrees of hydration, provided that the molar ratios between these components are respected. For example, the calcium acetate may also be used in anhydrous form or as hemihydrate or dihydrate and the aluminum sulfate can be used in anhydrous form or with a number of hydration water molecules variable from 6 to 27, particularly as hexadecahydrate, heptadecahydrate or octadecahydrate.

The aforementioned second object is achieved, in a first aspect, by providing a pharmaceutical formulation for topical application comprising, as active components, calcium acetate, preferably calcium acetate monohydrate, aluminum sulfate, preferably aluminum sulfate tetradecahydrate, and a further active component, selected from potassium permanganate (KMnO₄) and chloramine-T (sodium N-chloro-4-toluenesulfonamidate), and a pharmaceutically acceptable carrier.

In an aspect thereof, such a formulation comprises, for one part by weight of calcium acetate monohydrate, 1.41 parts of aluminum sulfate tetradecahydrate and from 0.01 to 0.4 parts of potassium permanganate.

In another aspect thereof, such a formulation comprises, for one part by weight of calcium acetate monohydrate, 1.41 parts of aluminum sulfate tetradecahydrate and from 0.025 to 0.75 parts of chloramine-T.

The formulation according to the invention is preferably in the form of lotion, emulsion, hydrophilic gel, cream, spray, foam, and transdermal patch. In the case of a cream formulation, the above active components are contained in the aqueous phase of the emulsion that constitutes the cream.

The formulation according to the invention can also be used as a detergent solution and shampoo.

The formulation according to the invention may contain one or more pharmaceutically acceptable adjuvants, such as dyes, fragrances, emollients, wetting agents, preservatives, protective colloids, vitamins, chelating agents, thickeners, gelling agents, antioxidants, film forming agents, photostabilizing agents, sunscreens, stabilizers, surfactants, viscosity modifiers, skin permeability enhancers, polymers and copolymers, pH regulators, pigments.

The pharmaceutically acceptable carriers that can be used in the topical formulations according to the present invention include, without limitation, water, glycerin, C₂-C₅ alcohols, fatty alcohols, fatty alcohol ethers, fatty acid esters, glycols, vegetable oils, mineral oils, liposomes, silicone oils and combinations thereof.

The carrier of the formulation according to the present invention can be in the form of aqueous phase, gel, oil in water emulsion, wax in water emulsion, silicone in water emulsion.

When the carrier is in the form of an aqueous phase, it may contain water only or a mixture of water and at least one organic solvent miscible with water, such as an alcohol having from 1 to 5 carbon atoms, and in particular ethanol or propanol, a polyol, e.g. propylen glycol, glycerol, diglycerol, panthenol, polyethylen glycol and mixtures thereof.

When the formulation according to the invention is in the form of a hydrophilic gel, gelling agents may be used selected from the group comprising carbomers, in particular carbomer 940, polyacrylamide, isoparaffin-laureth-7, xanthan gum, carrageenan, acacia gum, guar gum, agar gel, alginates, methylhydroxy cellulose, carboxymethyl cellulose, hydroxypropyl cellulose, hydroxypropyl methylcellulose, hydroxyethyl cellulose, ethyl cellulose, polyacrylates, polyvinyl alcohol, polyvinylpyrrolidone, colloidal silica.

When the formulation according to the invention is in the form of an emulsion, it may comprise a surfactant in a quantity variable from 0.1% to 30% by weight on the weight of the formulation.

When the formulation according to the invention is in the form of a cream or an emulsion in general, the relative lipophilic phase may comprise one or more oily components selected from the group comprising animal oils, triglycerides of fatty acids having from 4 to 10 carbon atoms, e.g. triglycerides of heptanoic or octanoic acid, vegetable oils such as sunflower, soybean, corn, grapeseed, castor, avocado oil, triglyceride caprylic/capric, jojoba oil, liquid paraffins, esters of fatty alcohols such as isopropyl myristate, isostearil isostearate, esters of pentaerythritol, fatty alcohols containing from 12 to 26 carbon atoms, linear or cyclic volatile and non-volatile silicone oils, for example cyclomethicones and dimethicones.

Among the antioxidants which may be included in the formulation according to the present invention it is mentioned in particular ascorbic acid and its derivatives, beta-carotene, catechins, curcumin, gallic acid and its derivatives, lycopene, rosmarinic acid, tannic acid, tocopherol and its derivatives, glutathione, lipoic acid, thioglycolic acid, bisulfites and metabisulphites, parabens, butyl hydroxyanisole and butyl hydroxytoluene.

The formulations according to the present invention may also contain a particulate phase comprising pigments or opacifiers, such as titanium dioxide, zinc oxide, zirconium oxide, iron oxide.

The formulations according to the present invention may further comprise at least one further active ingredient, selected from the group comprising steroidal and non-steroidal anti-inflammatory agents, antibiotics, antimycotics and local anesthetics.

The steroid anti-inflammatory agents contained in the formulation according to the invention are preferably selected from the group comprising: cortisol, cortisone, prednisone, prednisolone, methylprednisolone, meprednisolone, triamcinolone, paramethasone, fluprednisololone, betamethasone and dexamethasone.

The non-steroidal anti-inflammatory agents used in the formulation according to the invention are preferably selected from the group comprising: acetylsalicylic acid, diflunisal, ibufenac, diclofenac, indomethacin, sulindac, ibuprofen, naproxen, ketoprofen, fenoprofen, flurbiprofen, indoprofen, mefenamic acid, meclofenamic acid, flufenamic acid, niflumic acid, metamizole, paracetamol, piroxicam, meloxicam, tenoxicam, nimesulide, diflumidone, rofecoxib, celecoxib, suflamizole, tiflamizole, bromelain, serratiopeptidase, benzydamine.

The antibiotics contained in the formulation according to the invention are preferably selected from the group comprising: ampicillin, amoxicillin, carbenicillin, ticarcillin, mezlocillin, piperacillin, azlocillin, apalcillin and other penicillins, cefazolin, cephalexin, cephalothin, cephapirin, cefuroxime, cefonicid, cefaclor, cefoxitin, cefotetan, cefotaxime, ceftriaxone, cefixime and other cephalosporins, imipenem, meropenem, ertapenem, bacitracin, ciprofloxacin, levofloxacin, trovafloxacin, erythromycin, clarithromycin and azithromycin.

The local anesthetics contained in the formulation according to the invention are preferably selected from the group comprising: 2,4-dichlorobenzyl alcohol, benzocaine, benzyl benzoate, bupivacaine, lidocaine and mepivacaine.

The antimycotics contained in the formulation according to the invention are preferably selected from the group comprising: miconazole, econazole, fluconazole, clotrimazole, itraconazole, nystatin, terbinafine, griseofulvin, undecylenic acid and usnic acid.

The formulations according to the present invention can be prepared by techniques known to the expert of the field, described for example in Remington's Pharmaceutical Sciences, XXII Ed. Allen, Loyd V., Jr.

The compositions and formulations according to the present invention are endowed with an astringent effect and an enhanced antimicrobial action and are useful for the topical treatment of various diseases, including swelling, hives, eczemas, erythemas, insect bites, bruises, itching, vesicular or bullous exudation, crusted lesions, skin ulcers, atopic dermatitis, herpes zoster, herpes simplex, intertrigo and sunburn where there is or there may be present a microbial component which influences the evolution thereof.

In particular, it was observed for the compositions and formulations according to the present invention a synergistic effect in relation to their antimicrobial action. In particular, the active components of the composition according to the present invention, as will be demonstrated in the following detailed description, have shown to be able to exert, when combined according to the teachings of the present invention, an effective antimicrobial action at concentrations below their minimum inhibitory concentrations.

### Detailed description

Below some embodiments of the present invention will be set forth, for illustration and not limitation purposes.

### Example 1

### Sachets for the preparation of a solution containing aluminum acetate and potassium permanganate

| | |
|---|---|
| calcium acetate monohydrate | 0.991 g |
| aluminum sulfate tetradecahydrate | 1.403 g |
| potassium permanganate | 0.022 g |
| dextrin | 0.300 g |

The sachets are prepared starting from 2.416 g of a powder mixture obtained by homogeneously mixing one part by weight of calcium acetate monohydrate with 1.41 parts by weight of aluminum sulfate tetradecahydrate and 0.02 parts of potassium permanganate. To these 2.416 g of the powder mixture, 0.300 g of dextrin are added and the whole is further mixed until homogeneity.

By diluting the content of a sachet in 0.5 1 of water, an opalescent solution is obtained containing 0.16% of aluminum acetate and 0.0044% of potassium permanganate.

If two or three sachets in 0.5 1 of water are used, concentrations of aluminum acetate equal to 0.32% and 0.48% and concentrations of potassium permanganate equal to 0.0088%, and 0.0132% will be respectively obtained.

### Example 2

### Sachets for the preparation of a solution containing aluminum acetate and chloramine-T.

| | |
|---|---|
| calcium acetate monohydrate | 0.991 g |
| aluminum sulfate tetradecahydrate | 1.403 g |
| chloramine-T | 0.026 g |

The sachets are prepared from 2.420 g of a powder mixture obtained by homogeneously mixing one part by weight of calcium acetate monohydrate with 1.41 parts by weight of aluminum sulfate tetradecahydrate and 0.025 parts of chloramine-T.

By diluting the content of a sachet in 0.51 of water, an opalescent solution is obtained containing 0.16% of aluminum acetate and 0.0052% of chloramine-T.

If two or three sachets in 0.5 liters of water are used, concentrations of aluminum acetate equal to 0.32% and 0.48% and concentrations of chloramine-T equal to 0.0104% and 0.0156% will be respectively obtained.

### Example 3

### Hydroalcoholic gel (% by weight on the total weight)

| | |
|---|---|
| calcium acetate monohydrate | 1.00 |
| aluminum sulfate tetradecahydrate | 1.41 |
| chloramine-T | 0.19 |
| carbomer | 1.50 |
| ethanol 96° | 31.56 |
| essential oils | q.s. |
| pur. water | q.s. to 100 g |

### Example 4

| Cream (% by weight on the total weight) | |
|---|---|
| calcium acetate monohydrate | 1.00 |
| aluminum sulfate tetradecahydrate | 1.41 |
| chloramine-T | 0.09 |
| glyceryl monostearate | 8.00 |
| macrogol cetostearyl ether | 2.5 |
| liquid paraffin | 2.0 |
| white vaseline | 2.0 |
| isostearil isostearate | 4.0 |
| myristyl alcohol | 3.0 |
| parabens | 0.3 |
| pur. water | q.s. to 100 g |

### Example 5

| Cream (% by weight on the total weight) | |
|---|---|
| calcium acetate monohydrate | 1.00 |
| aluminum sulfate tetradecahydrate | 1.41 |
| potassium permanganate | 0.02 |
| propylene glycol | 20.00 |
| oleic acid | 5.00 |
| macrogol stearate | 9.00 |
| cetostearyl alcohol | 6.00 |
| dimethicone | 0.30 |
| carbopol 980 | 0.30 |
| trometamol | 0.10 |
| sodium sulfite | 0.10 |
| butyl hydroxy anisole | 0.02 |
| pur. water | q.s. to 100 g |

### Example 6

| Gel (% by weight on the total weight) | |
|---|---|
| diethylene glycol monoethylether | 43.00 |
| ethanol 96° | 29.00 |
| polyoxyl 40 hydrogenated castor oil | 5.00 |
| camphor | 3.00 |
| hydroxypropyl cellulose | 2.00 |
| calcium acetate monohydrate | 1.00 |
| aluminum sulfate tetradecahydrate | 1.41 |
| chloramine-T | 0.10 |
| essential oils | 1.00 |
| methyl salicylate | 0.80 |
| pur. water | q.s. to 100 g |

### Example 7

| Anti-dandruff shampoo (% by weight on the total weight) | |
|---|---|
| sodium lauryl ether sulfate 70% | 16.00 |
| coco betaine aqueous sol. 30% | 6.00 |
| calcium acetate monohydrate | 1.00 |
| aluminum sulfate tetradecahydrate | 1.41 |
| chloramine-T | 0.10 |
| preservatives | q.s. |
| pur. water | q.s. to 100 g |

### Example 8

The synergistic effect of the composition according to the present invention in relation to its antimicrobial activity was evaluated with the method of the checkerboard technique, as described by Hsieh MH et al⁴.

The "checkerboard" method encompasses the assessment of the microbial growth in wells containing different concentrations of two compounds to be tested, corresponding to different fractions of the minimum inhibitory concentration of each of the two compounds.

The microbial strains on which the test was ran were prepared and the minimum inhibitory concentrations (MICs) were determined as described by White RL et al⁵ (for S. *aureus*, *P. aeruginosa*), by Chen Y-L et al⁶ (for *C. Albicans*), by Perrins N et al⁷ (for *T. mentagrophytes*) and by Sopirala MM et al.⁸ (for A. *baumannii*).

The test allowed the calculation of an index (FIC index) derived from the sum of the ratio of the inhibitory concentrations of the test substances dilutions in combination and the respective MICs, which was interpreted as follows: synergy defined by an FIC index ≤ 0.5; indifference from an index > 0.5 and ≤ 4; antagonism in case of FIC Index > 4 (see Hsieh MH et al⁴).

The obtained results are reported in the following Tables 1 and 2.

**Table 1**

| Species | FIC index of aluminum acetate plus | | | |
|---|---|---|---|---|
| | Potassium permanganate | Chloramine -T | Benzalkonium chloride | Chlorhexidine |
| *S. aureus* | 0.5 (S) | 0.5 (S) | 1 (I) | 2 (I) |
| *P. aeruginosa* | 0.25 (S) | 0.5 (S) | 1 (I) | 2(I) |
| *C. albicans* | 0.25 (S) | 0.25 (S) | 2 (I) | 1 (I) |
| *A. baumannii* | 0.25 (S) | 0.25 (S) | 1 (I) | 1 (I) |
| *T. mentagrophytes* | 0.25 (S) | 0.5 (S) | 2 (I) | 1 (I) |

| | | | | |
|---|---|---|---|---|
| (S) = synergy, (I) = indifference | | | | |

The aluminum acetate used for the test was obtained by appropriate dilution of a stock solution prepared by dissolving 0.991 g of calcium acetate monohydrate and 1.403 g of aluminum sulfate tetradecahydrate in 0.5 1 of water.

As can be seen from the Table 1, the combination of aluminum acetate and potassium permanganate as well as the combination of aluminum acetate and chloramine-T have clearly shown a synergistic antimicrobial action against the tested microorganisms where no synergistic action was found for the aluminum acetate/benzalkonium chloride and aluminum acetate/ chlorhexidine combinations.

**Table 2**

| Species | FIC index of potassium permanganate plus | | |
|---|---|---|---|
| | Chloramine-T | Benzalkonium chloride | Chlorhexidine |
| *S. aureus* | 0.25 (S) | 2 (I) | 1 (I) |
| *P. aeruginosa* | 0.5 (S) | 1 (I) | 1 (I) |
| *C. albicans* | 0.5 (S) | 2 (I) | 2 (I) |
| *A. baumannii* | 0.25 (S) | 1 (I) | 1 (I) |
| *T. mentagrophytes* | 0.5 (S) | 1 (I) | 1 (I) |

Also in this case it has been demonstrated a clear synergistic antimicrobial action for the potassium permanganate/chloramine-T combination but not for the potassium permanganate/benzalkonium chloride and potassium permanganate/chlorhexidine combinations.

### Example 9

The compositions according to the present invention were studied in vivo to evaluate their effect on the timing of repair of skin wounds. For this evaluation the model described in Dovi JV et al⁹ was used that encompasses the evaluation of re-epithelialization percentage, as a function of time, after shaving.

In addition to the treatment as described by the authors, after shaving, the following formulations were used, by means of 5 washes per day for a lasting period of 3 minutes:
AC = aluminium acetate solution at the minimum inhibitory concentration, obtained by dilution of a solution of 0.991 g of calcium acetate monohydrate and 1.403 g of aluminum sulfate tetradecahydrate in 0.5 1 of water.
PP = potassium permanganate solution at the MIC, obtained by dilution of a solution of 0.040 g of potassium permanganate in 0.5 1 of water.
CT = chloramine-T solution at the MIC, obtained by dilution of a 0.300 g of chloramine-T solution in 0.5 1 of water.
E1 = solution obtained by mixing (parts by volume) 1 part of AC solution with 1 part of PP solution and diluting the whole with 3 parts of water.
E2 = solution obtained by mixing (parts by volume) 1 part of AC solution with 1 part of CT solution and diluting the whole with 3 parts of water.
E3 = solution obtained by mixing (parts by volume) 1 part of PP solution with 1 part of CT solution and diluting the whole with 3 parts of water (not according to the invention).

The results are shown in Table 3.

**Table 3**

| | Time | | | | |
|---|---|---|---|---|---|
| | Day 1 | Day 2 | Day 3 | Day 4 | Day 5 |
| AC | 10% | 40% | 60% | 75% | 100% |
| PP | 12% | 38% | 58% | 76% | 100% |
| CT | 9% | 41% | 63% | 74% | 100% |
| E1 | 20% | 65% | 100% | 100% | 100% |
| E2 | 18% | 63% | 100% | 100% | 100% |
| E3 (not according to the invention) | 20% | 59% | 100% | 100% | 100% |

The application of the solutions E1, E2 and E3 has allowed a more rapid re-epithelialization of the skin after shaving with respect to the application of the solutions AC, PP and CT.

### Bibliographic References:

1. Thorp MA, Gardiner IB, Prescott CA. Burow's solution in the treatment of active mucosal chronic suppurative otitis media: determining an effective dilution. J Laryngol Otol. 2000 Jun; 114(6):432-6.
2. Thorp MA, Oliver SP, Kruger J, Prescott CA. Determination of the lowest dilution of aluminium acetate solution able to inhibit in vitro growth of organisms commonly found in chronic suppurative otitis media. J Laryngol Otol. 2000 Nov;114(11):830-1.
3. Food and Drug Administration, HHS. Astringent drug products that produce aluminum acetate; skin protectant drug products for over-the-counter human use; technical amendment. Final rule; technical amendment. Fed Regist. 2009 Mar 6;74(43):9759-65.
4. Hsieh MH, Yu CM, Yu VL, Chow JW. Synergy assessed by checkerboard. A critical analysis. Diagn Microbiol Infect Dis. 1993 May-Jun; 16(4):343-9.
5. White RL, Burgess DS, Manduru M, Bosso JA. Comparison of three different in vitro methods of detecting synergy: time-kill, checkerboard, and E test. Antimicrob Agents Chemother. 1996 Aug;40(8):1914-8.
6. Chen YL, Lehman VN, Averette AF, Perfect JR, Heitman J. Posaconazole exhibits in vitro and in vivo synergistic antifungal activity with caspofungin or FK506 against Candida albicans.
7. Perrins N, Howell SA, Moore M, Bond R. Inhibition of the growth in vitro of Trichophyton mentagrophytes, Trichophyton erinacei and Microsporum persicolor by miconazole and chlorhexidine. Vet Dermatol. 2005 Oct;16(5):330-3.
8. Sopirala MM, Mangino JE, Gebreyes WA, Biller B, Bannerman T, Balada-Llasat JM, Pancholi P. Synergy testing by Etest, microdilution checkerboard, and time-kill methods for pan-drug-resistant Acinetobacter baumannii. Antimicrob Agents Chemother. 2010 Nov;54(11):4678-83.
9. Dovi JV, He LK, DiPietro LA. Accelerated wound closure in neutrophil-depleted mice. J Leukoc Biol. 2003 Apr;73(4):448-55.

## Claims

1. A powder composition comprising calcium acetate, preferably calcium acetate monohydrate, aluminum sulfate, preferably aluminum sulfate tetradecahydrate, and a third component selected from potassium permanganate (KMnO₄) and chloramine-T (sodium N-chloro-4-toluenesulfonamidate) and optionally at least one pharmaceutically or cosmetically acceptable excipient.

2. The composition according to claim 1, comprising, in parts by weight, one part of calcium acetate monohydrate, 1.41 parts of aluminum sulfate tetradecahydrate and from 0.01 to 0.4 parts of potassium permanganate.

3. The composition according to claim 1, comprising, in parts by weight, one part of calcium acetate monohydrate, 1.41 parts of aluminum sulfate tetradecahydrate and from 0.025 to 0.75 parts of chloramine-T.

4. The composition according to claim 1 or 2, consisting of calcium acetate monohydrate, aluminum sulfate tetradecahydrate and a third component selected from potassium permanganate and chloramine-T.

5. The composition according to any one of claims 1 to 4 for use in the preparation of an aqueous dispersion containing at least 0.065% w/w of aluminum acetate, useful for the topical treatment of skin conditions selected from the group comprising swelling, hives, eczemas, erythemas, insect bites, bruises, itching, vesicular or bullous exudation, crusted lesions, skin ulcers, atopic dermatitis, herpes zoster, herpes simplex, intertrigo and sunburn.

6. A pharmaceutical formulation for topical application comprising, as active components, calcium acetate, preferably monohydrate, aluminum sulfate, preferably tetradecahydrate, and a further active component selected from potassium permanganate (KMnO₄) and chloramine-T (sodium N-chloro-4-toluenesulfonamidate), and a pharmaceutically acceptable carrier.

7. The formulation according to claim 6 comprising, per one part by weight of calcium acetate monohydrate, 1.41 parts of aluminum sulfate tetradecahydrate and from 0.01 to 0.4 parts of potassium permanganate.

8. The formulation according to claim 6 comprising, per one part by weight of calcium acetate monohydrate, 1.41 parts of aluminum sulfate tetradecahydrate and from 0.025 to 0.75 parts of chloramine-T.

9. The formulation according to any one of claims 6-8, **characterized in that** it is in the form of lotion, emulsion, hydrophilic gel, cream, spray, foam, transdermal patch, detergent solution or shampoo, and preferably comprising one or more pharmaceutically acceptable adjuvants, selected from the group comprising dyes, fragrances, emollients, wetting agents, preservatives, protective colloids, vitamins, chelating agents, thickeners, gelling agents, antioxidants, film forming agents, photostabilizing agents, sunscreens, stabilizers, surfactants, viscosity modifiers, skin permeability enhancers, polymers and copolymers, pH regulators and pigments, and optionally at least one further active component, selected from the group comprising steroidal and non-steroidal anti-inflammatory agents, antibiotics, antimycotics and local anesthetics.

10. The formulation according to any one of claims 6-9 for use in the topical treatment of skin conditions selected from the group comprising swelling, hives, eczemas, erythemas, insect bites, bruises, itching, vesicular or bullous exudation, crusted lesions, skin ulcers, atopic dermatitis, herpes zoster, herpes simplex, intertrigo and sunburn.

## Patentansprüche

1. Pulverzusammensetzung umfassend Calciumacetat, bevorzugt Calciumacetatmonohydrat, Aluminiumsulfat, bevorzugt Aluminiumsulfattetradecahydrat und eine dritte Komponente ausgewählt unter Kaliumpermanganat (KMnO₄) und Chloramin-T (Natrium-N-chlor-4-toluolsulfonamidat) und wahlweise mindestens einen pharmazeutisch oder kosmetisch akzeptablen Trägerstoff.

2. Zusammensetzung nach Anspruch 1, umfassend, in Gewichtsteilen, einen Teil Calciumacetatmonohydrat, 1,41 Teile Aluminiumsulfattetradecahydrat und 0,01 bis 0,4 Teile Kaliumpermanganat.

3. Zusammensetzung nach Anspruch 1, umfassend, in Gewichtsteilen, einen Teil Calciumacetatmonohydrat, 1,41 Teile Aluminiumsulfattetradecahydrat und 0,025 bis 0,75 Teile Chloramin-T.

4. Zusammensetzung nach Anspruch 1 oder 2, bestehend aus Calciumacetatmonohydrat, Aluminiumsulfattetradecahydrat und einer dritten Komponente ausgewählt unter Kaliumpermanganat und Chloramin-T.

5. Zusammensetzung nach einem der Ansprüche 1 bis 4 zur Verwendung bei der Herstellung einer wässrigen Dispersion enthaltend mindestens 0,065 Gew.-/Gew.% Aluminiumacetat, die zur topischen Behandlung von Hautleiden ausgewählt aus der Gruppe umfassend Schwellung, Nesselausschlag, Hautekzemen, Erythemen, Insektenstichen, Prellungen, Juckreiz, blasenförmiger oder billöser Exsudation, verkrusteten Läsionen, Hautgeschwüren, atopischer Dermatitis, Gürtelrose, Herpes simplex, Intertrigo und Sonnenbrand nützlich ist.

6. Pharmazeutische Formulierung für die topische Anwendung umfassend, als aktive Komponenten, Calciumacetat, bevorzugt -monohydrat, Aluminiumsulfat, bevorzugt -tetradecahydrat und eine weitere aktive Komponente ausgewählt unter Kaliumpermanganat (KMnO₄) und Chloramin-T (Natrium-N-chlor-4-toluolsulfonamidat) und einen pharmazeutisch akzeptablen Träger.

7. Formulierung nach Anspruch 6, umfassend, pro Gewichtsteil Calciumacetatmonohydrat, 1,41 Teile Aluminiumsulfattetradecahydrat und 0,01 bis 0,4 Teile Kaliumpermanganat.

8. Formulierung nach Anspruch 6 umfassend, pro Gewichtsteil Calciumacetatmonohydrat, 1,41 Teile Aluminiumsulfattetradecahydrat und 0,025 bis 0,75 Teile Chloramin-T.

9. Formulierung nach einem der Ansprüche 6 - 8, **dadurch gekennzeichnet, dass** sie in Form einer Lotion, Emulsion, eines hydrophilen Gels, einer Creme, eines Sprays, Schaums, transdermalen Pflasters, einer Detergenslösung oder eines Shampoos vorliegt und bevorzugt ein oder mehrere pharmazeutisch akzeptable Hilfsmittel ausgewählt aus der Gruppe bestehend aus Farbstoffen, Duftstoffen, Linderungsmitteln, Benetzungsmitteln, Konservierungsmitteln, Schutzkolloiden, Vitaminen, Chelatbildnern, Verdickungsmitteln, Gelbildungsmitteln, Antioxidationsmitteln, Filmbildungsmitteln, Photostabilisiermitteln, Sonnenschutzmitteln, Stabilisatoren, Tensiden, Viskositätsmodifiziermitteln, Hautdurchdringbakeitsverbesserungsmitteln, Polymeren und Copolymeren, pH-Wert-Regulatoren und Pigmenten, und wahlweise mindestens eine weitere aktive Komponente ausgewählt aus der Gruppe bestehend aus steroidalen und nichtsteroidalen Antiinflammatorika, Antibiotika, Antimykotika und örtlichen Betäubungsmitteln umfasst.

10. Formulierung nach einem der Ansprüche 6-9 zur Verwendung bei der topischen Behandlung von Hautleiden ausgewählt aus der Gruppe bestehend aus Schwellung, Nesselausschlag, Hautekzemen, Erythemen, Insektenstichen, Prellungen, Juckreiz, blasenförmiger oder billöser Exsudation, verkrusteten Läsionen, Hautgeschwüren, atopischer Dermatitis, Gürtelrose, Herpes simplex, Intertrigo und Sonnenbrand.

## Revendications

1. Composition de poudre comprenant de l'acétate de calcium, de préférence de l'acétate de calcium monohydraté, du sulfate d'aluminium, de préférence du sulfate d'aluminium tétradécahydraté, et un troisième composant choisi parmi le permanganate de potassium (KMnO₄) et la chloramine-T (N-chloro-4-toluènesulfonamidate de sodium) et facultativement au moins un excipient pharmaceutiquement ou cosmétiquement acceptable.

2. Composition selon la revendication 1, comprenant, en parties en poids, une partie d'acétate de calcium monohydraté, 1,41 partie de sulfate d'aluminium tétradécahydraté et de 0,01 à 0,4 partie de permanganate de potassium.

3. Composition selon la revendication 1, comprenant, en parties en poids, une partie d'acétate de calcium monohydraté, 1,41 partie de sulfate d'aluminium tétradécahydraté et de 0,025 à 0,75 partie de chloramine-T.

4. Composition selon la revendication 1 ou 2, consistant en de l'acétate de calcium monohydraté, du sulfate d'aluminium tétradécahydraté et un troisième composant choisi parmi le permanganate de potassium et la chloramine-T.

5. Composition selon l'une quelconque des revendications 1 à 4 pour une utilisation dans la préparation d'une dispersion aqueuse contenant au moins 0,065 % p/p d'acétate d'aluminium, utile pour le traitement topique d'affections de la peau choisies dans le groupe comprenant gonflements, urticaires, eczémas, érythèmes, piqûres d'insectes, contusions, démangeaisons, exsudation vésiculeuse ou bulleuse, lésions formant une croûte, ulcères de la peau, dermatite atopique, herpès zoster, herpès simplex, intertrigo et coups de soleil.

6. Formulation pharmaceutique pour application topique comprenant, en tant que composants actifs, de l'acétate de calcium, de préférence monohydraté, du sulfate d'aluminium, de préférence tétradécahydraté, et un autre composant actif choisi parmi le permanganate de potassium (KMnO₄) et la chloramine-T (N-chloro-4-toluènesulfonamidate de sodium), et un support pharmaceutiquement acceptable.

7. Formulation selon la revendication 6, comprenant, pour une partie en poids d'acétate de calcium monohydraté, 1,41 partie de sulfate d'aluminium tétradécahydraté et de 0,01 à 0,4 partie de permanganate de potassium.

8. Formulation selon la revendication 6, comprenant, pour une partie en poids d'acétate de calcium monohydraté, 1,41 partie de sulfate d'aluminium tétradécahydraté et de 0,025 à 0,75 partie de chloramine-T.

9. Formulation selon l'une quelconque des revendications 6 à 8, **caractérisée en ce qu'**elle se trouve sous la forme de lotion, émulsion, gel hydrophile, crème, pulvérisation, mousse, patch transdermique, solution détergente ou shampooing, et de préférence comprenant un ou plusieurs adjuvants pharmaceutiquement acceptables, choisis dans le groupe comprenant des colorants, parfums, émollients, agents de mouillage, conservateurs, colloïdes protecteurs, vitamines, agents chélateurs, épaississants, gélifiants, antioxydants, agents filmogènes, agents photostabilisants, écrans solaires, stabilisants, agents tensioactifs, modificateurs de viscosité, agents d'amélioration de la perméabilité cutanée, polymères et copolymères, régulateurs de pH et pigments, et facultativement au moins un autre composant actif, choisi dans le groupe comprenant des agents anti-inflammatoires stéroïdiens et non stéroïdiens, antibiotiques, antimycotiques et anesthésiques locaux.

10. Formulation selon l'une quelconque des revendications 6 à 9 pour une utilisation dans le traitement topique d'affections de la peau choisies dans le groupe comprenant gonflements, urticaires, eczémas, érythèmes, piqûres d'insectes, contusions, démangeaisons, exsudation vésiculeuse ou bulleuse, lésions formant une croûte, ulcères de la peau, dermatite atopique, herpès zoster, herpès simplex, intertrigo et coups de soleil.
